# EUROPÄISCHE PATENTSCHRIFT

(11) **EP 2 089 084 B1**
(45) Veröffentlichungstag und Bekanntmachung des Hinweises auf die Patenterteilung: **24.02.2016**
(21) Anmeldenummer: 07856592.6
(22) Anmeldetag: 12.12.2007
(51) Int. Cl.: A61M 5/28, A61M 5/31, A61M 39/28

(54) **AUFSATZ FÜR EINE SPRITZE ODER EINE KARPULE**
ATTACHMENT FOR A SYRINGE OR A CARTRIDGE
GARNITURE POUR SERINGUE OU AMPOULE

(30) Priorität: 13.12.2006 DE 102006058719
(43) Veröffentlichungstag der Anmeldung: 19.08.2009
(73) Patentinhaber: Arzneimittel GmbH Apotheker Vetter & Co. Ravensburg, 88212 Ravensburg (DE)
(72) Erfinder: GLOCKER, Joachim, 88250 Weingarten (DE)
(74) Vertreter: Gleiss, Alf-Olav
(86) Internationale Anmeldenummer: PCT/EP2007/010844
(87) Internationale Veröffentlichungsnummer: WO 2008/071403

(56) Entgegenhaltungen:
- WO-A-2005/039669
- DE-A1- 2 415 496
- US-A- 4 243 034
- US-A- 5 489 274
- US-A1- 2005 215 952

## Beschreibung

Die Erfindung betrifft einen Aufsatz für eine Spritze oder eine Karpule mit einem Dichtelement gemäß Oberbegriff des Anspruchs 1. Aufsätze der hier angesprochenen Art sind bekannt. Sie dienen dazu, den Innenraum der Spritze oder Karpule dicht abzuschließen und gegen Verunreinigungen zu schützen. Bekannt ist es, eine Durchstechmembran zum Verschließen von Spritzen oder Karpulen unter anderem in Zusammenhang mit einem Aufsatz der hier angesprochenen Art zu verwenden. Es hat sich gezeigt, dass beim Durchstechen der Membran Partikel losgerissen werden können, die in das Innere der Spritze beziehungsweise Karpule gelangen und gegebenenfalls mit dem Inhalt der Spritze/Karpule einem Patienten verabreicht werden.

Aufgabe der Erfindung ist es daher, einen Aufsatz zu schaffen, der diesen Nachteil vermeidet.

WO2005/039669 und US5489274 beschreiben einen Aufsatz für eine Spritze mit einer Betätigungseinrichtung, die in einer ersten Funktionsstellung eine Kraft auf eine Wandung eines Durchlasses der Spritze derart ausübt, dass der Durchlass geschlossen ist und in einer zweiten Funktionsstellung den Durchlass offen lässt. Zur Lösung der genannten Aufgabe schlägt die Erfindung einen Aufsatz vor, der die in Anspruch 1 genannten Merkmale umfasst. Er weist ein Dichtelement mit einem Durchlass auf, über den eine Flüssigkeit in die Spritze oder Karpule eingebracht oder aus dieser ausgetragen werden kann. Der Aufsatz zeichnet sich durch eine Betätigungseinrichtung aus, die mit dem Dichtelement zusammenwirkt und den in diesem vorhandenen Durchlass verschließt oder freigibt. Dies geschieht dadurch, dass die Betätigungseinrichtung in einer ersten Funktionsstellung eine Kraft auf die Wand des Durchlasses derart ausübt, dass dieser verschlossen ist. In einer zweiten Funktionsstellung wird keine Kraft oder zumindest eine nur so geringe Kraft auf die Wand ausgeübt, dass der Durchlass offen ist. Das Dichtelement weist einen konischen oder zylindrischen Abschnitt auf, durch den der Durchlass verläuft. Die Betätigungseinrichtung weist einen Durchlasskanal auf, der einen auf den konischen Abschnitt einwirkenden Betätigungsbereich aufweist, wobei der Betätigungsbereich durch eine konische Aufweitung des Durchlasskanals gebildet wird. Der Durchlasskanal fluchtet mit dem Durchlass im Dichtelement.

Es zeigt sich also, dass das Dichtelement so ausgebildet ist, dass zur Herstellung eines Durchlasses zum Innenraum der Spritze oder Karpule ein Durchstechen des Materials des Dichtelements nicht erforderlich ist. Vielmehr wird ein Durchlass durch eine Betätigungseinrichtung verschlossen oder offen gelassen. Es ist also unmöglich, dass von dem Dichtelement irgendwelche Partikel abgesprengt werden, die in das Innere der Spritze oder Karpule gelangen und damit mit einer Injektionsflüssigkeit in einen Patienten gelangen können.

Besonders bevorzugt wird ein Ausführungsbeispiel des Aufsatzes, bei dem die Betätigungseinrichtung und das Dichtelement so ausgelegt sind, dass das Öffnen und Schließen des Durchlasses wiederholbar ist.

Weitere Ausgestaltungen ergeben sich aus den übrigen Unteransprüchen.

Die Erfindung wird im Folgenden anhand der Zeichnungen näher erläutert. Es zeigen:
- Figur 1: einen Längsschnitt durch ein erstes Ausführungsbeispiel eines Aufsatzes mit geschlossenem Durchlass;
- Figur 2: einen Längsschnitt durch das Ausführungsbeispiel gemäß Figur 1 mit offenem Durchlass;
- Figur 3: einen Längsschnitt durch ein zweites Ausführungsbeispiel eines Aufsatzes mit geschlossenem Durchlass und
- Figur 4: einen Längsschnitt durch das Ausführungsbeispiel gemäß Figur 3 mit offenem Durchlass.

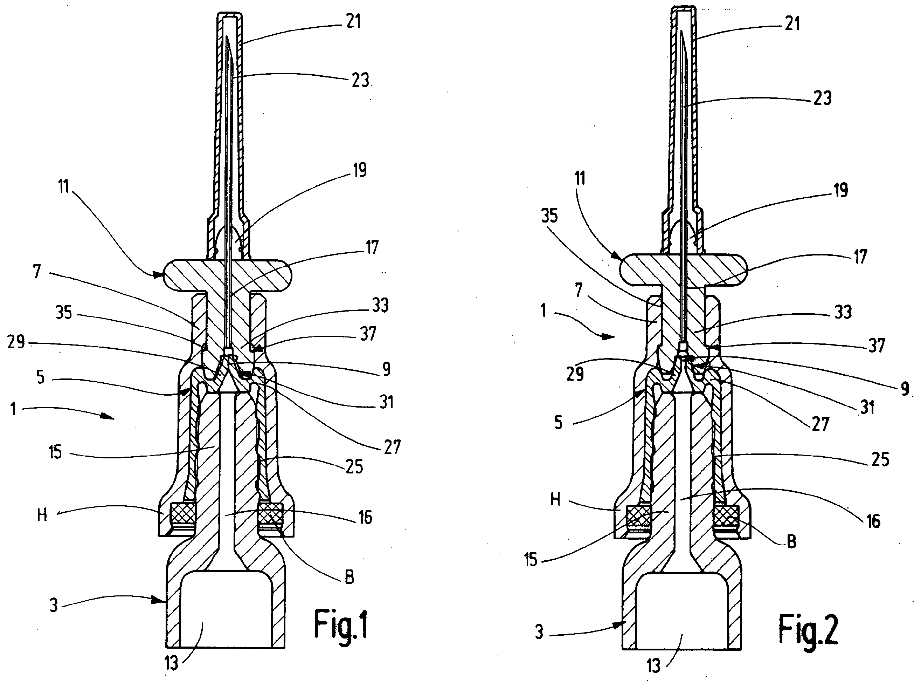

Figur 1 zeigt einen Aufsatz 1 für eine Spritze 3 mit einem Dichtelement 5, das im Inneren des Grundkörpers 7 des hohl ausgebildeten Aufsatzes 1 untergebracht ist und einen Durchlass 9 umfasst. Der Aufsatz 1 weist eine Betätigungseinrichtung 11 auf, die mit dem Dichtelement 5 zusammenwirkt und sich hier in einer ersten Funktionsstellung befindet, in der der Durchlass 9 im Dichtelement 5 verschlossen ist. Der Innenraum 13 der Spritze 3 ist damit dicht abgeschlossen.

Das hier dargestellte Ausführungsbeispiel der Spritze 3 weist einen Ansatz 15 auf, auf den der Aufsatz 1 aufgesetzt ist. Der Ansatz 15 ist innen hohl, sodass der Innenraum 13 der Spritze 3 über einen Zugang 16 in Fluidverbindung steht mit dem Durchlass 9 im Dichtelement 5. Dadurch, dass der Durchlass 9 verschlossen ist, ist der Innenraum 13 hermetisch abgeschlossen und gegen Verunreinigungen geschützt. Das Dichtelement 5 besteht vorzugsweise aus einem thermoplastischen Elastomer (TPE), das eine hohe Sicherheit gegen mikrobiologische Verunreinigungen bietet.

Die Betätigungseinrichtung 11 weist einen Durchlasskanal 17 auf, der mit dem Durchlass 9 fluchtet. Beispielhaft ist hier auf der der Spritze 3 abgewandten Oberseite der Betätigungseinrichtung 11 ein Ansatz 19 vorgesehen, auf den eine Kappe 21 für eine Kanüle 23 aufgesetzt ist, die zumindest bereichsweise in den Durchlasskanal 17 des Betätigungselements 11 eingesetzt, vorzugsweise eingeklebt ist. Denkbar ist es auch, die Kanüle 23 in den Grundkörper der Betätigungseinrichtung 11 einzuformen oder mittels eines Kunststoffspritgußverfahrens zu befestigen. Es sei hier ausdrücklich darauf hingewiesen, dass der Aufsatz 1 mit einem beliebigen medizinischen Anschluss versehen werden kann, dass die Kanüle 23 und die Kappe 21 hier also nur beispielhaft wiedergegeben sind.

Bei dem hier dargestellten Ausführungsbeispiel ist der Grundkörper 7 des Aufsatzes 1 hülsenförmig ausgebildet. Er umgreift lediglich den Ansatz 15 und nicht die Spritze 3 an sich. Es sei aber auch darauf hingewiesen, dass die Anbringung des Aufsatzes 1 an der Spritze 3 frei wählbar ist. Entscheidend ist hier, dass das Dichtelement 5 den Innenraum 13 der Spritze abschließen kann. Hier ist vorgesehen, dass der Aufsatz 1 kappenförmig ausgebildet ist und ein Dichtelement 5 aufnimmt, das hier vorzugsweise kappenförmig ausgebildet ist. Dieses kann in den Grundkörper 7 des Aufsatzes 1 eingesetzt oder eingespritzt sein. Es weist einen leicht konischen Bereich 25 auf sowie einen Dichtbereich 27, der den Zugang 16 zum Innenraum 13 der Spritze 3 abschließen kann. Hier in diesem Dichtbereich 27 ist der Durchlass 9 vorgesehen, und zwar bei dem Ausführungsbeispiel gemäß Figur 1 in einem konischen Abschnitt 29, der sich nach oben, also in Richtung der Betätigungseinrichtung 11 als Konus erhebt, also eine kegelmantelförmige Außenwand aufweist.

Die Betätigungseinrichtung 11 wirkt auf den konischen Abschnitt 29 des Dichtelements 5 dergestalt ein, dass von außen auf diesen Abschnitt eine Kraft ausgeübt wird, sodass die Wandung des Durchlasses 9 nach innen verlagert und der Durchlass 9 damit verschlossen wird. Dazu weist die Betätigungseinrichtung 11 einen Betätigungsbereich 31 auf, der hier durch eine konische Aufweitung des Durchlasskanals 17 gebildet wird. Die Innenkontur der konischen Aufweitung ist so auf die Außenkontur des konischen Abschnittes 29 abgestimmt, dass die Aufweitung flächig an dem konischen Abschnitt 29 anliegt und damit das Material des Dichtelements 5 nach innen drängt und den Durchlass 9 verschließt. Der Betätigungsbereich 31 kann auch durch einen zylindrischen Bereich mit einer Stufe gebildet werden, die von außen auf den konischen Abschnitt 29 drücken und damit den Durchlass 9 verschließen kann.

Die Betätigungseinrichtung 11 weist ein gegenüber dem Grundkörper 7 des Aufsatzes 1 verschiebbares Betätigungselement 33 auf, das koaxial zum Durchlass 9 angeordnet, in Richtung einer gedachten Mittelachse des Durchlasses 9 gegenüber dem Grundkörper 7 des Aufsatzes 1 verlagerbar ist und das den Betätigungsbereich 31 aufweist.

Figur 1 zeigt die Betätigungseinrichtung 11 in einer ersten Funktionsstellung. In dieser ist das Betätigungselement 33 ganz nach unten, das heißt in Richtung auf das Dichtelement 5 verschoben, sodass die konische Aufweitung des Durchlasskanals 17, also der Betätigungsbereich 31, von außen eine Druckkraft auf den konischen Abschnitt 29 des Dichtbereichs 27 ausübt und den Durchlass 9 verschließt. Das Betätigungselement 33 ist beispielsweise zylindrisch ausgebildet und greift durch eine Ausnehmung 35 im Grundkörper 7 hindurch. Vorzugsweise ist das Betätigungselement 33 zylindrisch ausgebildet, ebenso die Ausnehmung 35.

Es ist hier eine Verriegelungseinrichtung 37 vorgesehen, die die Betätigungseinrichtung 11 in ihrer in Figur 1 dargestellten Position verriegelnd hält. Mit dem Begriff verriegelnd ist hier gemeint, dass das Betätigungselement 33 nicht ohne weiteres, insbesondere nicht ungewollt, aus der in Figur 1 dargestellten ersten Funktionsstellung herausbewegt werden kann, sodass der Durchlass 9 sicher verschlossen ist. Beispielsweise ist auf der Außenseite des Betätigungselements 33 mindestens eine Nase vorgesehen, die in eine Vertiefung oder Nut in der Innenfläche der Ausnehmung 35 eingreift, sodass das Betätigungselement 33 in der ersten Funktionsstellung sicher gehalten wird.

Figur 2 zeigt das in Figur 1 dargestellte Ausführungsbeispiel des Aufsatzes 1 in einer zweiten Funktionsstellung. Gleiche Teile sind mit gleichen Bezugsziffern versehen, sodass auf deren Beschreibung verzichtet werden kann.

Das Betätigungselement 33 ist gegen die Kraft der Verriegelungseinrichtung 37 hier in eine zweite Funktionsstellung verlagert worden, in der es vorzugsweise wiederum verriegelnd gehalten wird. Die Betätigungseinrichtung 11, insbesondere deren Betätigungselement 33 ist in Richtung der Mittelachse des Durchlasses 9 nach oben verlagert worden, sodass die Innenwand der konischen Aufweitung des Durchlasskanals 17 hier in einem Abstand zur Außenfläche des konischen Abschnitts 29 des Dichtbereichs 27 des Dichtelements 5 liegt. Dadurch kann sich der Durchlass 9, wie in Figur 2 dargestellt, öffnen.

Die zweite Funktionsstellung der Betätigungseinrichtung 11 beziehungsweise des Betätigungselements 33 ist so gewählt, dass die von der konischen Aufweitung von außen auf den konischen Abschnitt 29 wirkende Kraft so gering ist, dass sich der Durchlass 9 öffnen kann. Es ist also nicht zwingend erforderlich, dass die Innenwand der konischen Aufweitung in einem Abstand zur Außenfläche des konischen Abschnitts 29 angeordnet ist.

Das Material des Dichtelements 5 ist so gewählt, dass der Durchlass 9 aufgrund der Eigenelastizität des konischen Abschnitts 29 nach außen federt, wenn die Innenwand der konischen Aufweitung des Durchlasskanals 17 auf den konischen Abschnitt 29 keine oder nur eine geringe Kraft ausübt.

Aufgrund der Eigenelastizität des Dichtelements 5 kann der konische Abschnitt 29 von dem Betätigungselement 33 mehrfach in seine geschlossene Position gedrängt werden, wie in Figur 1 dargestellt. Jedes Mal wenn die Betätigungseinrichtung 11 in ihre zweite Funktionsstellung gemäß Figur 2 verlagert wird, wenn also das Betätigungselement 33 nach oben verschoben wird, kann sich der konische Abschnitt 29 des Dichtbereichs 27 aufweiten und den Durchlass 9 freigeben. Es zeigt sich also, dass der Aufsatz 1 auf einfache Weise wiederverschließbar ist und dass bei der Eröffnung eines Zugangs zum Innenraum 13 der Spritze 3 keine Membran durchstochen werden muss, aus der Partikel losgerissen werden und einen Patienten gefährden könnten.

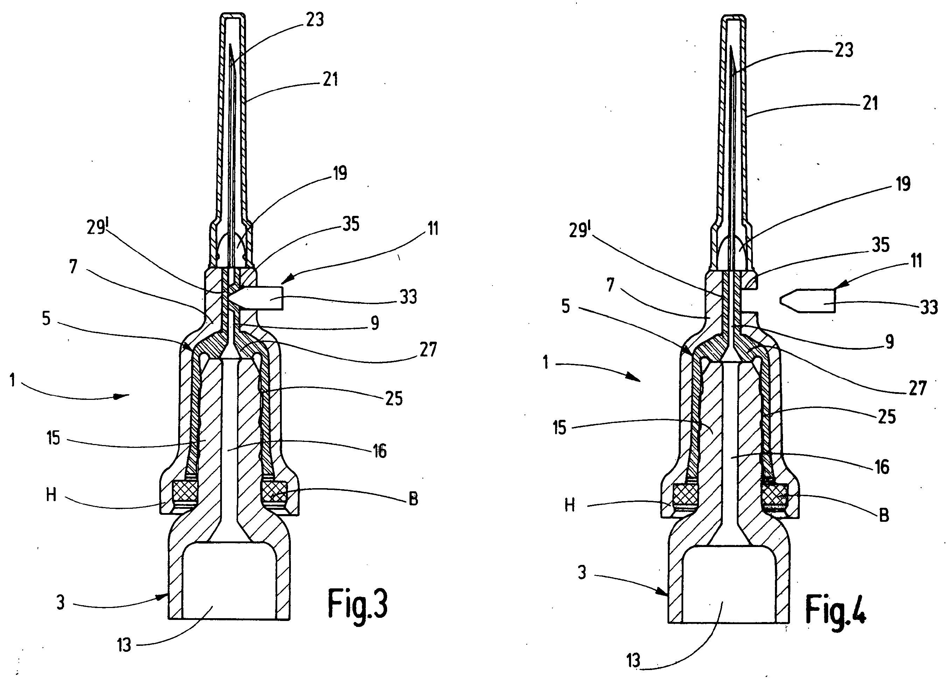

Figur 3 zeigt einen Längsschnitt durch ein zweites Ausführungsbeispiel eines Aufsatzes 1 mit einem geschlossenen Durchlass 9. Teile, die mit denen in den Figuren 1 und 2 übereinstimmen oder funktionsgleich sind, werden mit gleichen Bezugsziffern versehen.

Figur 3 zeigt einen Aufsatz 1, der auf eine Spritze 3 aufgebracht ist und ein Dichtelement 5 einschließt. Beispielhaft ist der Aufsatz 1 hier wiederum auf einen Ansatz 15 mit einem Zugang 16 aufgesetzt, der zum Innenraum 13 der Spritze 3 führt und mit einem Durchlass 9 im Dichtelement 5 fluchtet. Dieses umschließt mit einem zylindrischen Bereich 25 den Ansatz 15 und weist einen Dichtbereich 27 auf.

Bei dem hier dargestellten Ausführungsbeispiel weist der Dichtbereich 27 einen zylindrischen Abschnitt 29' auf, der ebenfalls von dem Durchlass 9 durchzogen wird, der im Übrigen mit dem Zugang 16 fluchtet.

Es zeigt auch hier, dass das Dichtelement 5 vorzugsweise auch als Dichtscheibe ausgebildet sein könnte, von welcher der zylindrische Abschnitt 29' dann ausginge.

An dem der Spritze 3 abgewandten oberen Ende des Grundkörpers 7 des Aufsatzes 1 ist ein Ansatz 19 für eine Kappe 21 vorgesehen, die eine Kanüle 23 umschließt. Auch hier ist festzuhalten, dass die Kanüle lediglich beispielhaft wiedergegeben ist. Es kann hier ein beliebiger medizinischer Anschluss vorgesehen werden.

Auch hier ist eine Betätigungseinrichtung 11 vorhanden, die ein Betätigungselement 33 umfasst, welches auf den Durchlass 9 einwirkt. Hier ist allerdings vorgesehen, dass das Betätigungselement 33 nicht, wie in Figur 1 und 2 dargestellt, in Richtung der Mittelachse des Durchlasses 9 verlagerbar ist, sondern seitlich auf den zylindrischen Abschnitt 29' einwirkt und damit eine Kraft auf die Wandung des Durchlasses 9 ausübt, sodass dieser bei der in Figur 3 dargestellten ersten Funktionsstellung des Betätigungselements 33 verschlossen ist.

Die Betätigungseinrichtung 11 wirkt durch eine Ausnehmung 35 im Grundkörper 7 des Aufsatzes 1 auf den zylindrischen Abschnitt 29' des Dichtbereichs 27 des Dichtelements 5 ein.

Figur 4 zeigt das in Figur 3 wiedergegebene Ausführungsbeispiel des Aufsatzes 1, wobei hier die Betätigungseinrichtung 11 in ihrer zweiten Funktionsstellung angeordnet ist, in der das Betätigungselement 33 keine Kraft von außen auf den zylindrischen Abschnitt 29' ausübt, vielmehr in einem Abstand zu diesem angeordnet ist. Es wäre sehr wohl auch denkbar, dass das Betätigungselement 33 noch auf der Außenseite des zylindrischen Abschnitts 29' aufliegt. Entscheidend ist, dass die von dem Betätigungselement 33 ausgeübte Kraft so gering ist, dass die Wandung des Durchlasses 9 nicht eingedrückt wird, wie dies in Figur 3 dargestellt ist.

Aufgrund der Eigenelastizität des Dichtelements 5, das vorzugsweise auch hier aus TPE hergestellt ist, wird erreicht, dass der Durchlass 9 von dem Betätigungselement 33 unter Kraftwirkung geschlossen, also quasi abgequetscht werden kann, dass aber bei einer geringen Kraft des Betätigungselements 33 oder bei einem Abheben des Betätigungselements 33 von dem zylindrischen Abschnitt 29' sich der Durchlass 9 wieder öffnet. Es ist also ohne weiteres möglich, den Durchlass 9 mehrfach zu öffnen und zu verschließen, ohne dass es erforderlich wäre, eine Membran zu durchstoßen, um zum Innenraum 13 der Spritze 3 zu gelangen. Damit ist sichergestellt, dass auch hier keine Partikel von einer Membran herausgerissen werden, was zu einer Gefährdung eines Patienten führen könnte.

Das an seiner dem zylindrischen Abschnitt 29' zugewandten Seite konisch zugespitzte Ende des Betätigungselements 33 kann letztlich auch mehr oder weniger stumpf ausgebildet werden. Entscheidend ist die von außen auf den zylindrischen Abschnitt 29' wirkende Kraft, die die Wandung des Durchlasses 9 zusammendrückt und zu einem Verschluss führt. Es können auch zwei oder mehr auf den zylindrischen Abschnitt 29' wirkende Betätigungselemente vorgesehen werden, die in Längsrichtung des Durchlasses 9 zueinander versetzt oder in einer gedachten Ebene, auf der der Durchlass 9 senkrecht steht, gegenüberliegend oder sternförmig zum zylindrischen Abschnitt 29' angeordnet sind.

Die Betätigungseinrichtung 11 kann ein schwenkbares Betätigungselement 33 aufweisen, das seitlich durch die Ausnehmung 35 gegen den zylindrischen Abschnitt 29' geschwenkt wird. Denkbar ist es aber auch, in die Wandung des Grundkörpers 7 des Aufsatzes 1 eine Schraube einzuschrauben, die mehr oder weniger weit in Richtung auf den zylindrischen Abschnitt 29' eingeschraubt wird und dazu führt, dass der Durchlass 9 verschlossen oder freigegeben wird.

Denkbar ist es im Übrigen auch, das Betätigungselement 33 durch eine in Richtung des Durchlasses 9 verschiebbare Hülse mit einer mehr oder weniger großen Kraft nach innen gegen den zylindrischen Abschnitt 29' zu drücken, beispielsweise durch eine Schiebe- oder Gewindehülse, um den Durchlass 9 in einer ersten Funktionsstellung zu verschließen und in einer zweiten Funktionsstellung freizugeben.

Grundsätzlich gilt dies auch für das Ausführungsbeispiel gemäß den Figuren 1 und 2: Das dort beschriebene Betätigungselement 33 ist durch Zug- oder Druckkräfte axial, das heißt in Richtung des Durchlasses 9 verlagerbar. Es ist hier möglich, das Betätigungselement 33 als Schraube mit einem Betätigungsbereich 31 auszubilden, die mehr oder weniger weit in die Ausnehmung 35 eingeschraubt wird und damit auf den konischen Abschnitt 29 mit einer mehr oder weniger großen Kraft einwirkt und damit den Durchlass 9 verschließt oder freigibt.

Aus den Erläuterungen wird deutlich, dass der Aufsatz 1 auch unmittelbar auf den Grundkörper 7 der Spritze 3 aufsetzbar ist, dass also auf den Ansatz 15 verzichtet werden kann. Entscheidend ist die Ausgestaltung des Dichtelements 5 mit dem konischen Abschnitt 29 beziehungsweise dem zylindrischen Abschnitt 29'. Dabei kann das Dichtelement 5 sehr wohl auch scheibenförmig ausgebildet sein.

Bei den Figuren 1 bis 4 wurde beispielhaft ein Haltering H an dem der Spritze 3 zugewandten Ende des Aufsatzes 1 vorgesehen, der mit dem Grundkörper 7 des Aufsatzes 1 über eine Sollbruchlinie verbunden ist und der auf einen Befestigungsring B verriegelnd aufgesetzt ist, der an der Spritze 3 oder Karpule, hier am Ansatz 15 angebracht ist. Auf diese Ausgestaltung kann ebenfalls verzichtet werden.

Schließlich sei hier noch festgehalten, dass der Aufsatz 1 nicht nur mit einer Spritze 3, sondern auch mit einer Karpule verwendet werden kann.

Beiden Ausführungsbeispielen des Aufsatzes 1 nach den Figuren 1 und 2 beziehungsweise nach den Figuren 3 und 4 ist gemeinsam, dass der Durchlass 9 im Dichtelement 5, der eine Verbindung zum Innenraum 13 der Spritze 3 schafft, mehrfach geöffnet und verschlossen werden kann.

Es ist also möglich, den Inhalt einer Spritze 3 in mehreren Einzeldosen einem oder einer Anzahl von Patienten zu verabreichen. Zwischen jeder Applikation kann die Spritze 3 auf einfache Weise verschlossen werden, beispielsweise auch, um die Kanüle 23 auszutauschen.

Möglich ist es auch, die Spritze 3 mit einem Medium zu füllen und diese mit geöffnetem Durchlass 9 einem Gefriertrocknungsverfahren zuzuführen. Das fertige Lyophilisat kann dann sicher im Inneren einer Spritze oder Karpule eingeschlossen werden, indem der Durchlass 9 verschlossen wird.

Denkbar ist es beispielsweise auch, den Innenraum 13 der Spritze 3 mit einem Unterdruck zu beaufschlagen, die Kanüle 23 beispielsweise in eine Vene eines Patienten einzuführen und dann den Durchlass 9 zu öffnen, um durch den Unterdruck in der Spritze Blut zu entnehmen. Danach kann die Spritze auf einfache Weise mittels der Betätigungseinrichtung 11 wieder verschlossen werden, das heißt, der Durchlass 9 wird mittels des Betätigungselements 33 abgeschlossen.

## Patentansprüche

1. Aufsatz für eine Spritze oder eine Karpule mit einem Dichtelement (5), das einen Durchlass (9) für ein in der Spritze oder Karpule vorhandenes oder in die Spritze oder die Karpule einzubringendes Medium aufweist, wobei eine Betätigungseinrichtung (11) vorgesehen ist, die in einer ersten Funktionsstellung eine Kraft auf die Wandung des Durchlasses (9) derart ausübt, dass der Durchlass (9) verschlossen ist, und die in einer zweiten Funktionsstellung den Durchlass (9) offen lässt, **dadurch gekennzeichnet, dass** das Dichtelement (5) einen konischen Abschnitt (29) aufweist, durch den der Durchlass (9) verläuft, dass die Betätigungseinrichtung (11) einen Durchlasskanal (17) aufweist, der einen auf den konischen Abschnitt (29) einwirkenden Betätigungsbereich (31) aufweist, wobei der Betätigungsbereich (31) durch eine konische Aufweitung des Durchlasskanals (17) gebildet wird, und dass der Durchlasskanal (17) mit dem Durchlass (9) im Dichtelement (5) fluchtet.

2. Aufsatz nach Anspruch 1, **dadurch gekennzeichnet, dass** die Betätigungseinrichtung (11) und das Dichtelement (5) so ausgelegt sind, dass das Öffnen und Schließen des Durchlasses (9) wiederholbar ist.

3. Aufsatz nach Anspruch 1 oder 2, **dadurch gekennzeichnet, dass** das Dichtelement (5) elastisches Material aufweist, vorzugsweise aus diesem besteht.

4. Aufsatz nach Anspruch 3, **dadurch gekennzeichnet, dass** der Durchlass (9) durch die Eigenelastizität des Materials des Dichtelements (5) offen gehalten wird.

5. Aufsatz nach einem der vorhergehenden Ansprüche, **dadurch gekennzeichnet, dass** die Betätigungseinrichtung (11) ein Betätigungselement (33) aufweist, das gegenüber einem Grundkörper (7) des Aufsatzes (1) beweglich ist.

6. Aufsatz nach Anspruch 5, **dadurch gekennzeichnet, dass** eine Verriegelungseinrichtung (37) vorgesehen ist, die mit dem Betätigungselement (33) so zusammenwirkt, dass dieses in mindestens einer Position verriegelnd gehalten wird.

7. Aufsatz nach einem der Ansprüche 5 oder 6, **dadurch gekennzeichnet, dass** das Betätigungselement (33) in Richtung des Durchlasskanals (17) der Betätigungseinrichtung (11) verlagerbar ist.

## Claims

1. Attachment for a syringe or a cartridge with a sealing element (5), comprising an opening (9) for a medium in the syringe or cartridge or for a medium to be introduced into the syringe or the cartridge, wherein an actuating device (11) is provided, which exerts a force on the wall of the opening (9) in a first functional position in such a way that the opening (9) is closed, and which leaves the opening (9) open in a second functional position, **characterized in that** the sealing element (5) comprises a conical section (29), through which the opening (9) runs, that the actuating device (11) comprises a passage channel (17) having an actuating region (31) acting on the conical section (29), wherein the actuating region (31) is formed by a conical widening of the passage channel (17), and that the passage channel (17) is aligned with the opening (9) in the sealing element (5).

2. Attachment according to claim 1, **characterized in that** the actuating device (11) and the sealing element (5) are designed in such a way that opening and closing the opening (9) can be repeated.

3. Attachment according to claims 1 or 2, **characterized in that** the sealing element (5) comprises elastic material and is preferably made thereof.

4. Attachment according to claims 3, **characterized in that** the opening (9) is left open due to the inherent elasticity of the material of the sealing element (5).

5. Attachment according to one of the preceding claims, **characterized in that** the actuating device (11) comprises an actuating element (33) that can be moved in relation to a base body (7) of the attachment (1).

6. Attachment according to claims 5, **characterized in that** a locking device (37) is provided, interacting with the actuating element (33) in such a way that the actuating element is held in at least one position in a locking manner.

7. Attachment according to claim 5 or 6, **characterized in that** the actuating element (33) can be displaced in the direction of the passage channel (17) of the actuating device (11).

## Revendications

1. Embout pour une seringue ou une carpule avec un élément d'étanchéité (5) ayant un passage (9) pour un milieu qui est présent dans la seringue ou la carpule, ou peut être introduit dans la seringue ou la carpule, dans lequel est prévu un dispositif d'actionnement (11) qui applique, dans une première position fonctionnelle, une force sur le paroi du passage (9) de telle manière que le passage (9) est fermé, et, dans une seconde position fonctionnelle, laisse ouvert le passage (9), **caractérisée en ce que** l'élément d'étanchéité (5) présente une partie conique (29) à travers laquelle le passage (9) s'étend, **en ce que** le dispositif d'actionnement (11) présent une conduite de passage (17) ayant une zone d'actionnement (31) agissant sur la partie conique (29), la zone d'actionnement (31) étant constituée par un élargissement conique de la conduite de passage (17), et **en ce que** la conduite de passage (17) s'aligne avec le passage (9) dans l'élément d'étanchéité (5).

2. Embout selon la revendication 1, **caractérisée en ce que** le dispositif d'actionnement (11) et l'élément d'étanchéité (5) sont conçus de telle manière que l'ouverture et la fermeture du passage (9) sont répétables.

3. Embout selon la revendication 1 ou 2, **caractérisée en ce que** l'élément d'étanchéité (5) comprend du matériau élastique, et de préférence consiste en ce matériau.

4. Embout selon la revendication 3, **caractérisée en ce que** le passage (9) est maintenu ouvert au moyen de l'élasticité propre du matériau de l'élément d'étanchéité (5).

5. Embout selon l'une quelconque des revendications précédentes, **caractérisée en ce que** le dispositif d'actionnement (11) présent un élément d'actionnement (33) qui est mobile par rapport à un corps de base (7) du embout (1).

6. Embout selon la revendication 5, **caractérisée en ce qu**'il est prévu un dispositif de verrouillage (37) qui coopère avec l'élément d'actionnement (33) de telle manière que ce dernier est maintenu de façon verrouillante dans au moins une position.

7. Embout selon la revendication 5 ou 6, **caractérisée en ce que** l'élément d'actionnement (33) peut être déplacé dans la direction de la conduite de passage (17) du dispositif d'actionnement (11).
